(19) Europäisches Patentamt / European Patent Office / Office européen des brevets

(11) **EP 3 059 251 B1**

(12) **EUROPEAN PATENT SPECIFICATION**

(45) Date of publication and mention of the grant of the patent:
**03.04.2019 Bulletin 2019/14**

(21) Application number: **14854425.7**

(22) Date of filing: **14.10.2014**

(51) Int Cl.:
*C08B 1/00* (2006.01)  *B01J 2/06* (2006.01)
*B01J 20/285* (2006.01)  *B01J 20/24* (2006.01)
*B01J 20/30* (2006.01)  *C07K 1/22* (2006.01)
*C08B 15/10* (2006.01)  *C08J 9/16* (2006.01)
*C08J 9/28* (2006.01)  *G01N 30/52* (2006.01)
*G01N 30/88* (2006.01)  *B01D 15/38* (2006.01)

(86) International application number:
**PCT/JP2014/077361**

(87) International publication number:
**WO 2015/056680 (23.04.2015 Gazette 2015/16)**

(54) **METHOD FOR MANUFACTURING POROUS CELLULOSE BEADS**

VERFAHREN ZUR HERSTELLUNG VON PORÖSEN CELLULOSEPERLEN

PROCÉDÉ DE FABRICATION DE BILLES POREUSES EN CELLULOSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.10.2013 JP 2013215120**

(43) Date of publication of application:
**24.08.2016 Bulletin 2016/34**

(73) Proprietor: **Kaneka Corporation Osaka-shi, Osaka 530-8288 (JP)**

(72) Inventors:
• **HIRANO, Masaru**
  **Takasago-shi**
  **Hyogo 676-8688 (JP)**
• **WATANABE, Kana**
  **Settsu-shi**
  **Osaka 566-0072 (JP)**

(74) Representative: **Vossius & Partner Patentanwälte Rechtsanwälte mbB Siebertstrasse 3 81675 München (DE)**

(56) References cited:
WO-A1-2012/033223  WO-A1-2012/121258
CA-A1- 2 828 369  CN-A- 101 274 985
JP-A- H09 132 601  JP-A- 2011 231 152

• BOEDEN H F ET AL: "Bead cellulose derivatives as supports for immobilization and chromatographic purification of proteins", JOURNAL OF CHROMATOGRAPHY A, ELSEVIER, AMSTERDAM, NL, vol. 552, 9 August 1991 (1991-08-09), pages 389-414, XP026514558, ISSN: 0021-9673, DOI: 10.1016/S0021-9673(01)95956-4 [retrieved on 1991-08-09]

SEQUENCE LISTING

<110> KANEKA CORPORATION

<120> Method for producing porous cellulose beads

<130> Z1561 EP

<140> EP 14 85 4425.7
<141> 2014-10-14

<150> JP 2013-215120
<151> 2013-10-15

<160> 1

<170> PatentIn version 3.5

<210> 1
<211> 291
<212> PRT
<213> Staphylococcus aureus

<400> 1

```
Ala Gln His Asp Glu Ala Gln Gln Asn Ala Phe Tyr Gln Val Leu Asn
1               5               10                  15

Met Pro Asn Leu Asn Ala Asp Gln Arg Asn Gly Phe Ile Gln Ser Leu
            20              25                  30

Lys Asp Asp Pro Ser Gln Ser Ala Asn Val Leu Gly Glu Ala Gln Lys
        35              40                  45

Leu Asn Asp Ser Gln Ala Pro Lys Ala Asp Ala Gln Gln Asn Lys Phe
        50              55                  60

Asn Lys Asp Gln Gln Ser Ala Phe Tyr Glu Ile Leu Asn Met Pro Asn
65              70                  75                          80

Leu Asn Glu Glu Gln Arg Asn Gly Phe Ile Gln Ser Leu Lys Asp Asp
            85                  90                  95

Pro Ser Gln Ser Thr Asn Val Leu Gly Glu Ala Lys Lys Leu Asn Glu
        100             105                 110

Ser Gln Ala Pro Lys Ala Asp Asn Asn Phe Asn Lys Glu Gln Gln Asn
        115             120                 125

Ala Phe Tyr Glu Ile Leu Asn Met Pro Asn Leu Asn Glu Glu Gln Arg
    130             135                 140

Asn Gly Phe Ile Gln Ser Leu Lys Asp Asp Pro Ser Gln Ser Ala Asn
145             150                 155                         160

Leu Leu Ala Glu Ala Lys Lys Leu Asn Glu Ser Gln Ala Pro Lys Ala
            165                 170                 175

Asp Asn Lys Phe Asn Lys Glu Gln Gln Asn Ala Phe Tyr Glu Ile Leu
            180                 185                 190

His Leu Pro Asn Leu Asn Glu Glu Gln Arg Asn Gly Phe Ile Gln Ser
        195                 200                 205

Leu Lys Asp Asp Pro Ser Gln Ser Ala Asn Leu Leu Ala Glu Ala Lys
        210             215                 220

Lys Leu Asn Asp Ala Gln Ala Pro Lys Ala Asp Asn Lys Phe Asn Lys
225             230                 235                         240

Glu Gln Gln Asn Ala Phe Tyr Glu Ile Leu His Leu Pro Asn Leu Thr
            245             250                 255

Glu Glu Gln Arg Asn Gly Phe Ile Gln Ser Leu Lys Asp Asp Pro Ser
            260                 265                 270

Val Ser Lys Glu Ile Leu Ala Glu Ala Lys Lys Leu Asn Asp Ala Gln
        275             280                 285

Ala Pro Lys
        290
```

**Description**

TECHNICAL FIELD

The present invention relates to a method for producing porous cellulose beads.

BACKGROUND ART

Porous cellulose beads are safer than beads composed of a synthetic polymer, and the non-specific adsorption thereof is small. In addition, the mechanical strength of porous cellulose beads is high, and the beads have many hydroxy groups, which can be used for introducing a ligand capable of interacting with a target substance to be adsorbed. Accordingly, porous cellulose beads are used as a substrate material for various adsorbents such as an adsorbent for chromatography and an affinity adsorbent. Among the examples, an affinity adsorbent is used as a medical adsorbent and an adsorbent for purifying a medical antibody, since a target substance can be purified and an undesired substance amount can be reduced efficiently using an affinity adsorbent. In particular, as a medical adsorbent for treating rheumatism, hemophilia or dilated cardiomyopathy, an adsorbent produced by immobilizing Protein A as an affinity ligand on a porous carrier has attracted attention (for example, Non-patent document 1 and Non-patent document 2).

In addition, it has attracted attention that an adsorbent produced by immobilizing Protein A as an affinity ligand on a porous carrier is used as an adsorbent for purifying a medical antibody by specifically adsorbing an immune globulin, i.e. IgG.

Many methods for producing porous cellulose beads require a cumbersome step in comparison with the case of using a general synthetic polymer, since cellulose is considered to be hardly dissolved. As such a method, for example, Patent document 1 discloses a method in which cellulose is dissolved in a solvent such as calcium thiocyanate aqueous solution and coagulated. Such a solvent is highly corrosive and toxic, and it is difficult due to the solvent to design a plant. The cellulose solution used in the method shows peculiar behaviors, and the porous cellulose beads obtained by the method have considerably large pores and broad pore size distribution (for example, Non-patent document 3). When such porous cellulose beads obtained by the method are used for an adsorbent to adsorb an antibody and the like, high adsorption performance cannot be expected, since the specific surface area thereof is small. In addition, for example, Patent Document 2 discloses a method for producing a porous cellulose carrier by bonding a substituent group to the hydroxy group of cellulose in order to improve the solubility of the cellulose, dissolving the cellulose in a general solvent to carry out granulation, and then removing the substituent group. However, the steps of the method are cumbersome and molecular weight may be decreased during the steps of reacting and removing the substituent group. Thus, the strength of the beads tends to be not enough to be used in high-speed processing and large scale which have been recently required.

Furthermore, for example, Patent documents 3 and 4 disclose a method in which cellulose is dissolved in sodium hydroxide aqueous solution having low temperature. However, in the method described in Patent document 3, after the step of heating a mixture of cellulose and a hydrogen bond breaking agent at 100 to 350°C under pressure, the mixture is dissolved in an alkaline aqueous solution. Such a step is industrially disadvantageous. In addition, the method described in Patent document 4 requires the steps in which cellulose is dispersed in a strong base solution, and the dispersion is once frozen and then thawed.

Patent document 5 discloses the cellulose which can be dissolved in an alkaline solution. However, the cellulose is a micro fiber having a diameter of 1 μm or less, and further micronized to 500 nm or smaller. Such a micronizing procedure is not suitable for industrial production.

Very recently, Patent document 6 discloses a method in which microbial cellulose is dissolved in an alkaline solution to prepare a cellulose solution, the cellulose solution becomes particulate by adding a dispersion medium, and the microbial cellulose particle is frozen and then washed to obtain cellulose beads. However, the method is not suitable for an industrial production due to cumbersome steps.

Patent Document 7 relates to a method for producing porous cellulose beads, comprising the steps of: mixing a cold alkaline aqueous solution and cellulose to prepare a cellulose dispersion; and bringing the cellulose dispersion into contact with a coagulating solvent.

Patent Document 8 relates to a magnetic cellulose microsphere having a porous honeycomb structure on the surface, having a specific surface area of 100 to 450 $m^2/g$, a pore diameter of 200 to 800 nm, and a particle size of 1 to 600 μm.

PRIOR ART DOCUMENT

PATENT DOCUMENT

Patent Document 1: JP 2009-242770 A
Patent Document 2: WO 2006/025371
Patent Document 3: US 4634470 B
Patent Document 4: US 5410034 B
Patent Document 5: JP H9-124702 A
Patent Document 6: JP 2010-236975 A
Patent Document 7: CA 2 828 369 A1
Patent Document 8: CN 101 274 985 A

NON-PATENT DOCUMENT

Non-patent Document 1: Annals of the New York Academy of Sciences, Vol.1051, 2005, p.635-646
Non-patent Document 2: American Heart Journal, Vol.152, Number 4, 2006, p.712el-712e6
Non-patent Document 3: Journal of Chromatography, Vol.195, 1980, p.221-230

DISCLOSURE OF THE INVENTION

PROBLEMS TO BE SOLVED BY THE INVENTION

The objective of the present invention is to provide a method for easily and efficiently producing cellulose beads which have narrow pore size distribution and pore structure suitable for an adsorbent and of which adsorption performance is excellent without using highly toxic and highly corrosive auxiliary raw material and without industrially disadvantageous cumbersome step.

MEANS FOR SOLVING THE PROBLEMS

The inventors of the present invention made extensive studies to solve the above problems. As a result, the inventors completed the present invention by finding that porous cellulose beads of which pore size distribution is sharper and of which adsorption performance is more excellent when a ligand is immobilized on the beads can be efficiently produced by mixing an alkaline aqueous solution having low temperature and a cellulose powder to prepare a fine cellulose dispersion and adding a water-soluble low molecular organic compound to the fine cellulose dispersion.

The present invention is as defined in the claims. The present disclosure can be summarized by the following items:

[1] A method for producing porous cellulose beads, comprising

(a) the step of preparing a fine cellulose dispersion by mixing a low temperature alkaline aqueous solution and cellulose,
(b) the step of preparing a mixed liquid by adding a water-soluble low molecular organic compound to the fine cellulose dispersion,
(c) the step of preparing an emulsion by dispersing the mixed liquid in a dispersion medium,
(d) the step of contacting the emulsion with a coagulating solvent.

[2] The method according to the above [1], wherein the temperature of the alkaline aqueous solution in the step (a) is not less than 0°C and not more than 25°C.
[3] The method for producing porous cellulose beads according to the above [1] or [2], wherein the water-soluble low molecular organic compound is an amino acid.
[4] The method for producing porous cellulose beads according to the above [1] or [2], wherein the water-soluble low molecular organic compound is one or more amino acids selected from the group consisting of glycine, alanine, serine, threonine, asparagine, glutamine, aspartic acid, glutamic acid, lysine, arginine and histidine.

[5] The method for producing porous cellulose beads according to any one of the above [1] to [4], wherein the dispersion medium is one or more oil-soluble solvents selected from the group consisting of an animal and plant fat and oil, a hydrogenated animal and plant fat and oil, a fatty acid triglyceride, an aliphatic hydrocarbon solvent and an aromatic hydrocarbon.

[6] The method for producing porous cellulose beads according to any one of the above [1] to [5], wherein the coagulating solvent is an alcohol solvent or a mixed solvent of water and an alcohol solvent.

[7] An adsorbent, obtained by immobilizing a ligand capable of interacting with a target substance on the beads produced by the method for producing porous cellulose beads according to any one of the above [1] to [6].

[8] An adsorbent, comprising the porous cellulose beads produced by the method according to any one of the above [1] to [6] and a ligand capable of interacting with a target substance.

[9] A method for producing an adsorbent, comprising the step of immobilizing a ligand capable of interacting with a target substance on the porous cellulose beads produced by the method according to any one of the above [1] to [6].

[10] A purification method, comprising the step of using the adsorbent according to the above [7] or [8].

## EFFECT OF THE INVENTION

According to the present invention, cellulose beads which have narrow pore size distribution and pore structure suitable for an adsorbent and of which adsorption performance is excellent can be easily and efficiently produced without using highly toxic and highly corrosive auxiliary raw material and without industrially disadvantageous cumbersome step.

## BRIEF DESCRIPTION OF THE DRAWINGS

Fig. 1 is a SEM observation image of the magnified surface of the beads obtained in Example 1.
Fig. 2 is a SEM observation image of the magnified surface of the beads obtained in Comparative example 1.
Fig. 3 is a graph which demonstrates the pore size distribution of the beads obtained in Examples 3 to 5 and Comparative example 3.
Fig. 4 is a graph which demonstrates the pore size distribution of the beads obtained in Examples 3 and 6 and Comparative example 3.

## MODE FOR CARRYING OUT THE INVENTION

The method for producing porous cellulose beads according to the present invention is characterized in comprising (a) the step of preparing a fine cellulose dispersion by mixing a low temperature alkaline aqueous solution and cellulose, (b) the step of preparing a mixed liquid by adding a water-soluble low molecular organic compound which is an amino acid to the fine cellulose dispersion, (c) the step of preparing an emulsion by dispersing the mixed liquid in a dispersion medium, and (d) the step of contacting the emulsion with a coagulating solvent wherein the temperature of the alkaline aqueous solution in step (a) is not less than -20°C and not more than 10°C. The applicant of the present application has developed the invention to obtain porous cellulose by dispersing cellulose in sodium hydroxide aqueous solution having low temperature and contacting the dispersion with a coagulating solvent as WO 2012/121258 and others.

The present inventors found that when a water-soluble low molecular organic compound is added to a fine cellulose dispersion, an adsorbent having larger adsorption amount can surprisingly be obtained in comparison with an adsorbent prepared without adding a water-soluble low molecular organic compound, though the reason is not known. The present inventors consider that the reason is that a pore suitable for adsorption may be formed by preferably dispersing a water-soluble low molecular organic compound in a fine cellulose dispersion to form a micro region and transferring the water-soluble low molecular organic compound to a coagulating solvent or a washing solvent.

Hereinafter, the present invention method is described step by step.

Step (a): Step of preparing fine cellulose dispersion

In the present step, a low temperature alkaline aqueous solution and cellulose are mixed to prepare a fine cellulose dispersion.

In the present invention, the term "low temperature" means a temperature lower than an ordinary temperature. The low temperature may be lower than an ordinary temperature, and is

not less than -20°C since a temperature regulation equipment can be simple and a cost for regulating temperature can be low. In addition, as the low temperature is

10°C or lower, a cellulose dispersion is hardly discolored, and dispersibility and swellability of cellulose are improved. In the present disclosure, the low temperature is preferably not less than -10°C and not more than 20°C. When the temperature is -10°C or higher, an alkaline aqueous solution can be prevented from being frozen. On the one hand, when the temperature is 20°C or lower, a cellulose dispersion can be efficiently prepared and can be prevented from being colored. The temperature is more preferably not less than -5°C, even more preferably not less than -2°C, and particularly preferably not less than -1°C. The temperature is most preferably not less than 0°C in terms of the handling performance of water used for a cellulose dispersion and the easiness of temperature regulation. Furthermore, the temperature is more preferably not more than 9°C, not more than 5°C, not more than 4°C and not more than 1°C. In addition, the temperature of not more than 9°C is preferred, since the circularity of the obtained porous cellulose beads becomes higher.

An alkali to be used is not particularly restricted as long as an aqueous solution thereof exhibits alkalinity. In terms of availability, lithium hydroxide, sodium hydroxide and potassium hydroxide are preferred; and in terms of safety and price of a product, sodium hydroxide is most preferred.

The concentration of the alkali in the above-described alkaline aqueous solution is not particularly restricted, and is preferably not less than 3 wt% and not more than 20 wt%. When the concentration of the alkali is included in the range, the dispersibility and swellability of cellulose to the alkaline aqueous solution is preferably improved. The concentration of alkali is more preferably not less than 5 wt% and not more than 15 wt%, even more preferably not less than 7 wt% and not more than 10 wt%, and most preferably not less than 8 wt% and not more than 10 wt%.

The kind of the above-described cellulose is not particularly restricted. For example, it is not needed to use substituted cellulose such as a cellulose into which a substituent is introduced to improve solubility, and general unsubstituted cellulose can be used as a raw material, since cellulose may not be dissolved in the present invention method. However, a cellulose powder is preferably used as the cellulose in order to efficiently disperse cellulose in the alkaline aqueous solution.

The molecular weight of a raw material cellulose to be used is not particularly restricted, and the polymerization degree is preferably not more than 1000. When the polymerization degree is 1000 or less, the dispersibility and swellability of cellulose to the alkaline aqueous solution is preferably improved. In addition, when the polymerization degree is 10 or more, the mechanical strength of the obtained porous cellulose beads preferably becomes high. The polymerization degree is more preferably not less than 50 and not more than 500, even more preferably not less than 100 and not more than 400, particularly preferably not less than 200 and not more than 350, and most preferably not less than 250 and not more than 350.

The concentration of cellulose in the fine cellulose dispersion is not particularly restricted and appropriately adjusted, and for example, may be adjusted to not less than about 1 wt% and not more than about 20 wt%. The concentration is more preferably not less than 2 wt%, even more preferably not less than 5 wt%, and more preferably not more than 15 wt%, even more preferably not more than 10 wt%.

As a method for preparing the fine cellulose dispersion, an ordinary method can be employed. For example, a mixture of the alkaline aqueous solution and cellulose may be vigorously stirred with maintaining the temperature to be lowered.

Step (b): Step of preparing mixed liquid containing cellulose and water-soluble low molecular organic compound

Then, a water-soluble low molecular organic compound is added to the above-described fine cellulose dispersion to prepare a mixed liquid.

The above-described water-soluble low molecular organic compound to be used in the present invention is an amino acid.

In the present invention, the term "low molecular" means that a compound is not polymerized to have high molecular and for example, is not an organic resin. The water-soluble low molecular organic compound preferably has a molecular weight of not more than 1000. In the present invention, the term "water-soluble" means that a compound is dissolved in water without limitation or has relatively high solubility in water. Specifically, for example, when 1 g or 1 mL of an organic compound can be dissolved in not more than 30 mL of water at $20 \pm 5$°C, such a compound can be used. The amount of water is more preferably less than 10 mL, and even more preferably less than 1 mL.

As a specific water-soluble low molecular organic compound, an alcohol compound such as butanol and propanol; a polyol compound such as propylene glycol, glycerin, ethylene glycol and triethylene glycol; a sugar compound such as sucrose and fructose; a cellosolve compound such as methyl cellosolve and butyl cellosolve; a carbitol compound such as diethylene glycol monomethyl ether and diethylene glycol diethyl ether; an ether compound such as tetraethylene glycol dimethyl ether and 1,4-dioxane; an ester compound such as ethylene glycol monomethyl ether acetate; and the like are disclosed herein for reference.

The above-described water-soluble low molecular organic compound to be used in the present invention is preferably a solid at an ordinary temperature, since the adsorption amount becomes larger in such a case. Disclosed examples thereof include

an alkyl sulfate ester salt compound such as an alkyl sulfate ester salt and polyoxyethylene alkyl sulfate ester salt; a solid organic acid compound and a salt thereof, such as citric acid; a quaternary ammonium salt such as an alkyltrimethylammonium chloride and a dialkyldimethylammonium chloride, a vitamin comnound such as vitamin C and vitamin B. In the present invention, the low molecular organic compound is an amino acid or a salt thereof, such as glycine, alanine, serine, threonine, asparagine, glutamine, aspartic acid, glutamic acid, lysine, arginine and histidine.

It is more preferred that the above-described water-soluble low molecular organic compound has low toxicity in terms of safety of a product which is the case for

an amino acid.

In terms of availability at relatively low cost, glycine, alanine and lysine are preferred among an amino acid. Vitamin C and vitamin B are disclosed as examples of a vitamin compound.

An amount of the above-described water-soluble low molecular organic compound to be used is not particularly restricted and may be appropriately adjusted, and for example, the concentration of the above-described water-soluble low molecular organic compound in the above-described mixed liquid may be adjusted to not less than about 0.1 wt% and not more than about 5 wt%.

A method for adding the above-described water-soluble low molecular organic compound to the cellulose dispersion is not particularly restricted. For example, the water-soluble low molecular organic compound may be added to the cellulose dispersion, or the water-soluble low molecular organic compound may be added during the preparation of the cellulose dispersion. In addition, whether the water-soluble low molecular organic compound is in the form of a liquid or solid, the compound may be added as it is, a solution in which the compound is dissolved in a solvent may be added, or a dispersion or slurry of the compound may be added. The solvent and dispersion medium in such cases are not particularly restricted, and an organic solvent or water may be used. The temperature when the water-soluble low molecular organic compound is added is not particularly restricted, and is preferably not more than 25°C in order to prevent the beads from being colored.

It is not necessarily needed that the water-soluble low molecular organic compound is homogeneously dispersed or dissolved in the cellulose dispersion. When it is needed to homogeneously disperse or dissolve the compound, a procedure such as natural diffusion, stirring and shaking may be carried out.

As a method for preparing porous beads from the above-described mixed liquid, a publically-known granulation method described in WO 2012/121258 and others may be employed. In addition, a publically-known crosslinking method may be further applied to the porous beads.

Hereinafter, subsequent steps are briefly described.

Step (c): Step of preparing emulsion

In the present step, an emulsion is prepared by dispersing the above-described mixed liquid in a dispersion medium.

As the dispersion medium which constitutes of the emulsion, an animal and plant fat and oil, a hydrogenated animal and plant fat and oil, a fatty acid glyceride, an aliphatic hydrocarbon solvent and an aromatic hydrocarbon solvent are exemplified. In addition, a surfactant such as a non-ionic surfactant may be used.

An animal and plant fat and oil is exemplified by palm oil, shea butter, sal fat, illipe butter, lard, beef fat, canola oil, rice oil, peanut oil, olive oil, corn oil, soybean oil, perilla oil, cotton oil, sunflower oil, evening primrose oil, sesame oil, safflower oil, coconut oil, cacao oil, palm kernel oil, fish oil, wakame seaweed oil, kelp oil and the like. A hydrogenated animal and plant fat and oil is exemplified by palm hardened oil, palm extremely hardened oil, canola hardened oil, canola extremely hardened oil, soybean hardened oil, hardened oil of lard, hardened fish oil and the like. A fatty acid triglyceride may be any one of tri-, di- and mono-glyceride, and is exemplified by stearyl glyceride, palmitin glyceride, lauryl glyceride and the like. An aliphatic hydrocarbon solvent is exemplified by beeswax, candelilla wax, rice bran wax and the like. An aromatic hydrocarbon solvent is exemplified by benzene, toluene, chlorobenzene, dichlorobenzene and the like.

In order to prepare the emulsion, an appropriate amount of a surfactant may be added. Such a surfactant is exemplified by a sorbitan fatty acid ester such as sorbitan laurate, sorbitan stearate, sorbitan oleate, sorbitan trioleate and the like.

An amount of the dispersion medium to be used may be adjusted so that droplets of the above-described mixed liquid can be sufficiently dispersed. For example, the amount may be adjusted to one or more times by mass of the above-described mixed liquid. On the one hand, when the amount of the dispersion medium is excessively large, an amount of a waste liquid is excessively increased; therefore, the ratio is preferably not more than 10 times by mass. The ratio is more preferably not less than 2 times by mass, even more preferably not less than 4 times by mass, and not more than 8 times by mass, even more preferably not more than 7 times by mass, particularly preferably not more than 6 times by mass.

The emulsion may be prepared by an ordinary method. For example, the emulsion can be prepared by vigorously stirring a mixture of the above-described mixed liquid, the dispersion medium and the surfactant.

Step (d): Step of coagulation

Next, the porous cellulose beads are obtained by bringing the emulsion into contact with a coagulating solvent in order to extract the solvent from droplets of the fine cellulose dispersion.

The coagulating solvent is not particularly restricted as long as the coagulating solvent has an affinity for the solvent of the fine cellulose dispersion, and is exemplified by an alcohol solvent and a mixed solvent of water and an alcohol solvent. Such an alcohol solvent is exemplified by $C_{1-4}$ alcohol such as methanol, ethanol, n-propanol, isopropanol, n-butanol, isobutanol, s-butanol and t-butanol. For example, a ratio of water and alcohol in an alcohol aqueous solution may be adjusted to water : alcohol solvent = 80 : 20 to 5 : 95 by volume.

An amount of the coagulating solvent to be used is not particularly restricted and appropriately adjusted, and for example, may be adjusted to not less than about 20 v/w% and not more than about 150 v/w% to the above-described mixed liquid to be used.

A method for coagulation is not particularly restricted, and it is preferred that the coagulating solvent is added to the vigorously stirred emulsion so that droplets are not coalesced each other, since the emulsion is sometimes unstable.

After the coagulating solvent is added, the coagulated porous cellulose beads are separated by filtration, centrifuge or the like, and may be washed with water, an alcohol or the like. The obtained porous cellulose beads may be classified using a sieve or the like in order to control the particle size to be uniform.

Step (e): Step of crosslinking porous cellulose beads

It is preferred that the thus obtained porous cellulose beads are crosslinked to be crosslinked porous cellulose beads using a crosslinking agent in order to improve the strength.

The crosslinking agent has two or more reactive groups which are able to covalently bind to the hydroxy group on cellulose so as to crosslink cellulose molecules. The crosslinking condition and crosslinking agent in the present invention for crosslinked porous cellulose beads are not particularly restricted. For example, the method described in WO 2008/146906 can be used.

The crosslinking agent is exemplified by a halohydrin such as epichlorohydrin, epibromohydrin and dichlorohydrin; bisepoxide, i.e. bisoxirane, which has two functional groups; and polyepoxide, i.e. polyoxirane, which is multifunctional. Only one crosslinking agent may be used alone, or two or more crosslinking agents may be used in combination.

A solvent used in the reaction for crosslinking porous cellulose beads by a crosslinking agent may be appropriately selected, and is exemplified by a water-miscible organic solvent in addition to water. The example of such a water-miscible organic solvent includes an alcohol solvent such as methanol, ethanol and isopropanol, and a nitrile solvent such as acetonitrile. Two or more solvents may be mixed to be used for the crosslinking reaction.

The crosslinking reaction may be carried out multiple times, and the reaction solvent and the crosslinking agent may be changed in each time. For example, a first crosslinking reaction may be carried out in a water-miscible organic solvent, and a final crosslinking reaction may be carried out in water. In such a case, the solvent compositions from second to second last reactions may be the same as or different from that of a first reaction or a last reaction, or an intermediate composition between those of a first reaction and a last reaction. Alternatively, all of the reactions may be carried out in water. The conditions are also applied to the crosslinking agent. When the crosslinking reaction is carried out multiple times, it is preferred that the crosslinked porous cellulose is washed with water or the like to remove the crosslinking agent between the crosslinking reactions.

A base may be added to the reaction mixture in order to accelerate the crosslinking reaction. Such a base is exemplified by an alkali metal hydroxide such as sodium hydroxide and potassium hydroxide; an alkali metal hydrogencarbonate salt such as sodium hydrogencarbonate and potassium hydrogencarbonate; an alkali metal carbonate salt such as sodium carbonate and potassium carbonate; an organic base such as triethylamine and pyridine.

After the crosslinking reaction, the crosslinked porous cellulose beads may be washed with water or the like, since the beads is insoluble.

Step (f): Step of immobilizing ligand

An adsorbent can be obtained by immobilizing a ligand which interacts with a target substance on the porous cellulose beads obtained by the method according to the present invention. The adsorbent obtained by the present invention is less likely to exhibit non-specific adsorption; therefore, a pharmaceutical and a treatment with high safety can be provided and further, labor for an intermediate washing step can be saved as much as possible during purification and treatment by using the adsorbent.

In the present invention, the term "ligand" means an affinity ligand which has a specific affinity for a target substance to be purified on the adsorbent and which interacts with the target substance. More specifically, the target substance is an antibody, and the ligand is selected from an antigen, aprotein and a peptide fragment which specifically interact with the antibody.

The ligand has a specific affinity for a target substance which should be purified using the adsorbent.

A method for immobilizing a ligand on the porous cellulose beads is not particularly restricted, and an ordinary method may be employed. For example, various immobilization methods are exemplified, such as a method for immobilizing an amino group-containing ligand using a cyanogen bromide method, a trichlorotriazine method, an epoxy method, a tresyl chloride method, a periodic acid oxidation method, a divinylsulfonic acid method, a benzoquinone method, a carbonyldiimidazole method, an acyl azide method or the like; a method for immobilizing a hydroxy group-containing ligand using an epoxy method, a diazo coupling method or the like; a method for immobilizing a thiol group-containing ligand using an epoxy method, a tresyl chloride method, a divinylsulfonic acid method or the like; a method for immobilizing a carboxy acid group-containing ligand and a formyl group-containing ligand on an aminated carrier, as described in Kenichi KASAI et al., "Affinity chromatography" published by Tokyo Kagakudoujin, 1991, Table 8-1, Table 8-2 and Figure 8-15. The contents of the document are incorporated by reference herein.

The adsorbent can be used as an adsorbent for purification, particularly as an adsorbent for purifying an antibody pharmaceutical and medical adsorbent which have attracted attention in recent years. A ligand used for an adsorbent for purifying an antibody pharmaceutical is not particularly restricted, and is exemplified by an amino group-containing ligand such as an antigen and a protein which have specific affinity for an antibody; Protein A, Protein G, Protein L, and variants thereof; and a peptide having an antibody binding activity.

In particular, an adsorbent which is prepared by immobilizing Protein A, Protein G or a variant thereof as a ligand on a porous carrier has attracted attention as an adsorbent capable specifically adsorbing an immunoglobulin, i.e. IgG. The above-described Protein A used in the present invention is not particularly restricted, and natural Protein A, transgenic Protein A and the like may be used without restriction. In addition, a substance containing an antibody-binding domain, a variant thereof or an oligomer thereof, a fused protein and the like may be used. The polymerization number of such an oligomer may be not less than 2 and not more than 10. In addition, Protein A and the like to be used can be produced from an extract obtained from fungus body or a culturing supernatant by combining and/or repeating a purification method selected from a molecular weight fractionation, a fractional precipitation and the like in which various chromatography and membrane separation technique are utilized. Such a chromatography is exemplified by ionexchange chromatography, hydrophobic interaction chromatography, gel filtration chromatography and hydroxyapatite chromatography. In particular, it is preferred that Protein A is obtained by the method described in WO 2006/004067, US 5151350, WO 2003/080655, JP 2006-304633 A and WO 2010/110288. The adsorbent on which Protein A is immobilized can be also utilized as an adsorbent used for treating dilated cardiomyopathy and the like. In addition, the adsorbent on which dextran sulfate or the like is immobilized can be utilized as an adsorbent used for treating hypercholesterolemia.

A method for introducing a ligand on the porous cellulose beads may be selected from the above-described various immobilization methods, and it is more preferred that a reaction between a formyl group that a porous particle has and an amino group of a ligand is utilized to carry out immobilization. For example, the method described in JP H1-278534 A is used. All of the contents of the publication are incorporated by reference herein.

An amount of the ligand to be immobilized on the adsorbent is not particularly restricted, and for example, may be adjusted to not less than 1 mg and not more than 1000 mg per 1 mL of the porous cellulose beads. When the ratio is 1 mg or more, an adsorption amount of a target substance preferably becomes large. When the ratio is 1000 mg or less, the production cost may be preferably reduced. An amount of the ligand to be immobilized per 1 mL of the porous cellulose beads is more preferably not less than 2 mg, even more preferably not less than 4 mg, particularly preferably not less than 5 mg, and more preferably not more than 500 mg, even more preferably not more than 250 mg, particularly preferably not more than 200 mg, most preferably not more than 100 mg.

The use application of the adsorbent obtained according to the present invention is not particularly restricted, and the adsorbent is preferably used as a medical adsorbent. In particular, the adsorbent is preferably used as a therapeutic adsorbent for adsorbing a large-sized disease substance such as LDL cholesterol to be removed, since the surface porosity of the adsorbent is improved. In addition, the adsorbent can be used as various chromatographic carriers, particularly as an industrial chromatographic carrier which is used for filling a large-diameter column. In particular, when the adsorbent is used as an adsorbent for purifying an antibody pharmaceutical, of which demand has been very heavy recently, the effect of the adsorbent can be exhibited. In terms of the above points, the porous cellulose beads are preferably used for producing an adsorbent on which Protein A, Protein G or Protein L is immobilized.

A target substance can be purified by using the adsorbent obtained according to the present invention. Specifically, the adsorbent may be contacted with a solution of a target substance. A contacting method is not restricted, and the adsorbent

may be added to a solution which contains a target substance, or a target substance may be selectively adsorbed on the adsorbent according to the present invention by filling a column with the adsorbent as described above and flowing a solution containing the target substance through the column. In particular, when a column is filled with the adsorbent, a solution can be flowed at high speed so that a target substance can be efficiently purified, since the strength of the adsorbent is high.

Next, the adsorbent on which a target substance is selectively adsorbed is separated from a solution by filtration, centrifugation or the like. By such a step, a target substance can be separated from other substances. In addition, a target substances is separated from the

adsorbent by using an eluate. As such an eluate, for example, an acidic buffer solution of which pH value is not less than about 2.5 and not more than about 4.5 may be used.

EXAMPLES

Hereinafter, the example of the present invention is described. However, the present invention is not restricted to the following examples in any way. First, methods for evaluating the physical properties of produced porous cellulose beads are described.

Test example 1: SEM observation of beads surface

The porous cellulose beads obtained in each Production example and Reference example or adsorbent were washed with five times amount by volume of 30% ethanol to replace the liquid part contained in the porous cellulose beads by 30% ethanol. Then, the porous cellulose beads were similarly treated with 50% ethanol, 70% ethanol, 90% ethanol, special grade ethanol, special grade ethanol and special grade ethanol in turn in order to replace the liquid part by ethanol. Further, the porous cellulose beads were similarly treated by a mixed solvent of t-butyl alcohol / ethanol = 3 / 7. Next, the porous cellulose beads were treated by mixed solvents of t-butyl alcohol / ethanol = 5 / 5, 7 / 7, 9 / 1, 10 / 0, 10 / 0 and 10 / 0 in turns in order to replace the liquid part by t-butyl alcohol, and then freeze-dried. The freeze-dried beads were subjected to deposition treatment, and SEM image was photographed.

Test example 2: Measurement of dynamic adsorption amount at RT (Residence time) of 3 minutes

(1) Preparation of solutions

The following liquids were prepared.
Liquid A: phosphate buffer with a pH of 7.4 (manufactured by SIGMA)
Liquid B: 35 mM sodium acetate with a pH of 3.5, prepared from acetic acid (manufactured by NACALAI TESQUE, INC.), sodium acetate and reverse osmosis (RO) water
Liquid C: 1 M acetic acid prepared from acetic acid (manufactured by NACALAI TESQUE, INC.) and RO water
Liquid D: 1 mg/mL human polyclonal IgG solution, prepared from 1500 mg / 10 mL of "Gamma-globulin NICHIYAKU" (manufactured by NIHON PHARMACEUTICAL CO., LTD.) and Liquid A
Liquid E: 6 M urea prepared from urea (manufactured by KANTO CHEMICAL CO., INC.) and RO water

Each solution was deaerated before use.

(2) Filling and preparation

As a column chromatography apparatus, AKTAexplorer 100 (manufactured by GE Healthcare Biosciences Corporation) was used. A 22 μm mesh was attached to a column having a diameter of 0.5 cm and height of 15 cm, and 3 mL of the adsorbent sample prepared according to the present invention was added into the column. The column was filled with the adsorbent sample by flowing 20% ethanol aqueous solution prepared from ethanol (manufactured by Wako Pure Chemical Industries, Ltd.) and RO water at a linear speed of 450 cm/h for 1 hour. On a fraction collector, 15 mL correcting tubes were set. In the correcting tube for an eluent, a neutralizing liquid was preliminarily added.

(3) Purification of IgG

Through the above-described column, 9 mL of Liquid A was flowed at a linear speed of 300 cm/h and then Liquid D was flowed at a linear speed of 300 cm/h till 10% of IgG passed with monitoring UV. A loading amount of IgG when 5% of IgG passed through was determined to be 5% DBC at RT of 3 minutes. Next, after 30 mL of Liquid A was flowed at a linear speed of 300 cm/h, 30 mL of Liquid B was flowed at a linear speed of 300 cm/h to elute IgG. Then, 9 mL of Liquid C was flowed at a linear speed of 300 cm/h and 9 mL of Liquid E was flowed at a linear speed of 300 cm/h for recycling.

Test example 3: Quantitative determination of epoxy group

An epoxidized porous cellulose beads were filtered by suction on a glass filter ("3G-2" manufactured by TOP) for 15 minutes. After the suction-drying, 1.5 g of the porous cellulose beads were added into a screw tube (manufactured by Maruemu Corporation), and 4.5 mL of 1.3 M sodium thiosulfate aqueous solution prepared from sodium thiosulfate (manufactured by Wako Pure Chemical Industries, Ltd.) and RO water was added thereto. After the mixture was heated at 45°C for 30 minutes, RO water was added thereto so that the liquid amount became 50 mL. The mixture was transferred into 100 mL glass beaker, and several drops of 1% phenolphthalein solution prepared from phenolphthalein (manufactured by Wako Pure Chemical Industries, Ltd.) and ethanol were added thereto. The amount of epoxy group was measured by titration using 0.01 N hydrochloric acid (for quantitative analysis, manufactured by Wako Pure Chemical Industries, Ltd.).

Test example 4: Measurement of LDL cholesterol amount

To 100 mL of fresh blood obtained from a healthy subject, 500 $\mu$L of heparin was added and moderately mixed for anticoagulating treatment. The anticoagulated fresh blood obtained from a healthy subject was subjected to centrifugal treatment at 3000 rpm for 15 minutes to obtain blood plasma having a LDL cholesterol concentration of 70 mg/dL. The adsorbent was washed with normal saline solution. To 0.5 mL of the washed adsorbent, 3 mL of the blood plasma was added. The mixture was shaken at 37°C for 2 hours. After the shaking, an amount of LDL cholesterol in the supernatant was measured by using a LDL cholesterol kit ("Cholestest LDL" manufactured by SEKISUI MEDICAL CO., LTD.) in order to measure the amount of LDL cholesterol to be adsorbed on the adsorbent. In addition, an amount of LDL cholesterol in a solution prepared in a similar condition to the above-described condition except that equivalent amount of normal saline solution was used in place of the adsorbent was measured for comparison.

LDL cholesterol adsorption rate (%) = $[(C_0 - C_1) / C_0] \times 100$ wherein $C_0$ is a concentration of LDL cholesterol in an adsorption procedure using normal saline solution, $C_1$ is a concentration of LDL cholesterol in an adsorption procedure using an adsorbent.

Production example 1: Preparation of Protein A

The non-oriented type Protein A used in the present invention had an amino acid sequence of SEQ ID NO. 1. The Protein A corresponds to a part of the Protein A derived from Staphylococcus aureus other than S domain, i.e. signal sequence, and X domain, i.e. cell wall binding domain, and is described as SPA' in WO 2006/004067. The Protein A was prepared in accordance with Examples described in WO 2006/004067. The contents of WO 2006/004067 are incorporated by reference herein.

Example 1

(1) Preparation of alkaline aqueous solution

Using sodium hydroxide (manufactured by Wako Pure Chemical Industries, Ltd.) and distilled water, 33 wt% sodium hydroxide aqueous solution was prepared. The temperature thereof was adjusted to 4°C.

(2) Preparation of cellulose dispersion containing water-soluble low molecular organic compound

Into a separable flask, 70 g of distilled water and 9.3 g of cellulose were added. The mixture was stirred using two-stage rushton turbine blades at 150 to 200 rpm for 30 minutes until the temperature of the slurry became 4°C. Then, 41 g of 33 wt% sodium hydroxide aqueous solution which was cooled to 4°C was added thereto. The mixture was maintained with stirring at 500 rpm for 30 minutes. Next, 33 g of 6 wt% glycine aqueous solution was added as a water-soluble low molecular organic compound to the prepared cellulose dispersion. The mixture was stirred at 600 rpm for 15 minutes.

(3) Preparation of porous cellulose beads by liquid-liquid dispersion

To the above-described cellulose dispersion, 788 g of 1 wt% sorbitan monooleate solution in o-dichlorobenzene was added. The mixture was stirred at 4°C and 600 rpm for 15 minutes to disperse cellulose droplets, and 74 mL of methanol as a coagulating solvent was added thereto. The mixture was stirred at 4°C and 600 rpm for 30 minutes. Then, the solution was removed by filtration using a glass filter ("26G-3" manufactured by TOP), and the cellulose beads were washed to be obtained using 5 times volume of methanol and 5 times volume of distilled water in turns.

(4) Classification of porous cellulose beads

The obtained porous cellulose beads were subjected to wet classification using sieves of 38 $\mu$m and 90 $\mu$m.

(5) Crosslinking of porous cellulose beads

To 20 mL of the above-described classified porous cellulose beads, distilled water was added so that the volume was adjusted to 30 mL. The mixture was transferred into a reaction vessel. To the reaction vessel, 2.3 g of Denacol EX-314 (manufactured by Nagase ChemteX Corporation) containing glycerol polyglycidyl ether was added as a crosslinking agent. The mixture was heated to 40°C with stirring. After the temperature was adjusted to 40°C, the mixture was stirred for 30 minutes. Then, 7.1 mL of 2N NaOH aqueous solution was prepared from sodium hydroxide (manufactured by NACALAI TESQUE, INC.) and distilled water, and each 1/4 of the solution was added per 1 hour. During the addition, the temperature was maintained at 40°C with stirring. After the last 1/4 amount of the solution was added, the mixture was stirred at the same temperature for 1 hour. After the reaction, the beads were washed with 20 times or more volume of distilled water with suction-filtration to obtain first crosslinked beads. The same crosslinking reaction was repeated once more to obtain second crosslinked beads.

The obtained second crosslinked beads were transferred into a vessel, and distilled water was added thereto so that the total amount was adjusted to 10 times volume of the crosslinked porous cellulose beads. The mixture was heated using an autoclave at 120°C for 60 minutes. After the mixture was cooled to room temperature, the beads were washed with 5 times or more volume of distilled water as much as the beads to obtain the autoclaved second crosslinked beads.

(6) Preparation of adsorbent

An adsorbent on which Protein A was immobilized was prepared in accordance with the following procedures. To 11.0 mL of the crosslinked porous cellulose beads obtained in the above-described (5), RO water was added to adjust the total amount to 17.0 mL. The mixture was added into 50 mL centrifuge tube. The centrifuge tube was set on a mix rotor ("MIX ROTOR MR-3" manufactured by AS ONE Corporation) to stir the mixture. Then, 6.0 mL of 8.64 mg/mL sodium periodate aqueous solution was prepared by dissolving sodium periodate (manufactured by Wako Pure Chemical Industries, Ltd.) in RO water, and was added into the centrifuge tube containing the crosslinked porous cellulose beads. The mixture was stirred at 25°C for 1 hour. After the reaction, the beads were washed with RO water on a glass filter ("11GP100" manufactured by SIBATA SCIENTIFIC TECHNOLOGY LTD.) till the electrical conductivity of the filtrate became 1 $\mu$S/cm or lower to obtain formyl group-containing crosslinked porous cellulose beads. The electrical conductivity of the filtrate obtained by washing was measured using a conductivity meter ("ECTester10 Pure+" manufactured by EUTECH INSTRUMENTS). On a glass filter ("11GP100" manufactured by SIBATA SCIENTIFIC TECHNOLOGY LTD.), 9.0 mL of the obtained formyl group-containing crosslinked porous cellulose beads were put, and the liquid within the beads was replaced using 30 mL of buffer containing 0.5 M trisodium citrate dihydrate (manufactured by KANTO CHEMICAL CO., INC.) and 0.15 M sodium chloride (manufactured by KANTO CHEMICAL CO., INC.). After the replacement, the formyl group-containing crosslinked porous cellulose beads were added into a centrifuge tube and the total volume was adjusted to 14.0 mL using a buffer of pH 8 containing 0.5 M trisodium citrate dihydrate and 0.15 M sodium chloride.

Into the centrifuge tube, 5.327 g of 67.58 mg/mL solution of the Protein A produced in the above-described Production example 1 was added. Then, the pH value was adjusted to 12 using 0.08 N sodium hydroxide prepared from sodium hydroxide (manufactured by NACALAI TESQUE, INC.) and RO water, and the reaction was carried out at 6°C for 23 hours with stirring by a mixing rotor ("MIX ROTOR MR-3" manufactured by AS ONE Corporation). After the reaction for 23 hours, the pH of the reaction mixture was adjusted to 5.0 using 2.4 M citric acid prepared from citric acid (manufactured by KANTO CHEMICAL CO., INC.) and RO water. Then, the mixture was stirred at 6°C for 4 hours using a mixing rotor ("MIX ROTOR MR-3" manufactured by AS ONE Corporation). Next, 0.39 mL of 5.5% dimethylamine borane (DMAB) aqueous solution prepared from dimethylamine borane (manufactured by KISHIDA CHEMICAL Co., Ltd.) and RO water was added thereto, and the mixture was stirred at 6°C for 1 hour. Then, the reaction temperature was increased to 25°C, and the reaction was carried out at 25°C for 18 hours with stirring by a mixing rotor ("MIX ROTOR MR-3" manufactured by AS ONE Corporation). After the reaction, the amount of the immobilized Protein A was determined by measuring UV absorbance of absorption maximum at about 278 nm of the reaction mixture. The beads after the reaction was washed with RO water of which volume was threefold of the volume of the beads on a glass filter ("11GP100" manufactured by SIBATA SCIENTIFIC TECHNOLOGY LTD.). Then, threefold volume amount of 0.1 M citric acid aqueous solution prepared from citric acid monohydrate (manufactured by KANTO CHEMICAL CO., INC.) and RO water was added to the beads so that the total volume was adjusted to 30 mL or more. The mixture was added into a centrifuge tube and stirred at 25°C for 30 minutes to carry out acid washing.

After the acid washing, the beads were washed with RO water of which volume was threefold of the volume of the beads on a glass filter ("11GP100" manufactured by SIBATA SCIENTIFIC TECHNOLOGY LTD.). Next, threefold amount of an aqueous solution of 0.05 M sodium hydroxide and 1 M sodium sulfate prepared from sodium hydroxide (manufactured by NACALAI TESQUE, INC.), sodium sulfate (manufactured by KANTO CHEMICAL CO.,INC.) and RO water was added thereto. Then, an aqueous solution of 0.05 M sodium hydroxide and 1 M sodium sulfate was added so that the total volume was adjusted to 30 mL or more. The mixture was added into a centrifuge tube and stirred at room temperature for 30 minutes to carry out alkaline washing.

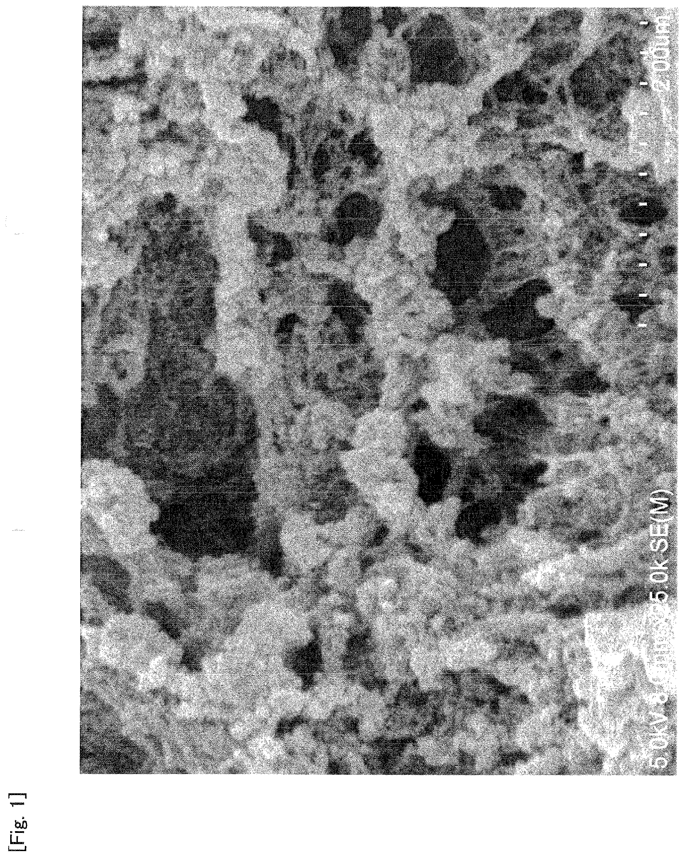

After the alkaline washing, the beads were washed with RO water of which volume was 20-fold of the volume of the beads on a glass filter ("11GP100" manufactured by SIBATA SCIENTIFIC TECHNOLOGY LTD.). Next, 0.5 N trisodium citrate aqueous solution prepared from trisodium citrate dihydrate (manufactured by KANTO CHEMICAL CO., INC.) and RO water of which volume was threefold of the volume of the beads was added. After it was confirmed that the filtrate became neutral, washing was carried out with RO water till the electrical conductivity of the filtrate became 1 $\mu$S/cm or lower to obtain the target adsorbent on which Protein A was immobilized. The electrical conductivity of the filtrate obtained by washing was measured using a conductivity meter ("ECTester10 Pure+" manufactured by EUTECH INSTRUMENTS). The physical properties of the obtained adsorbent were evaluated. As a result, the amount of the immobilized Protein A was 35 g per 1 L of the adsorbent, and the 5% DBC of the adsorbent at RT of 3 min was 39 g per 1 L of the filling adsorbent. The SEM observation image of the beads surface is shown as Figure 1.

Comparative example 1

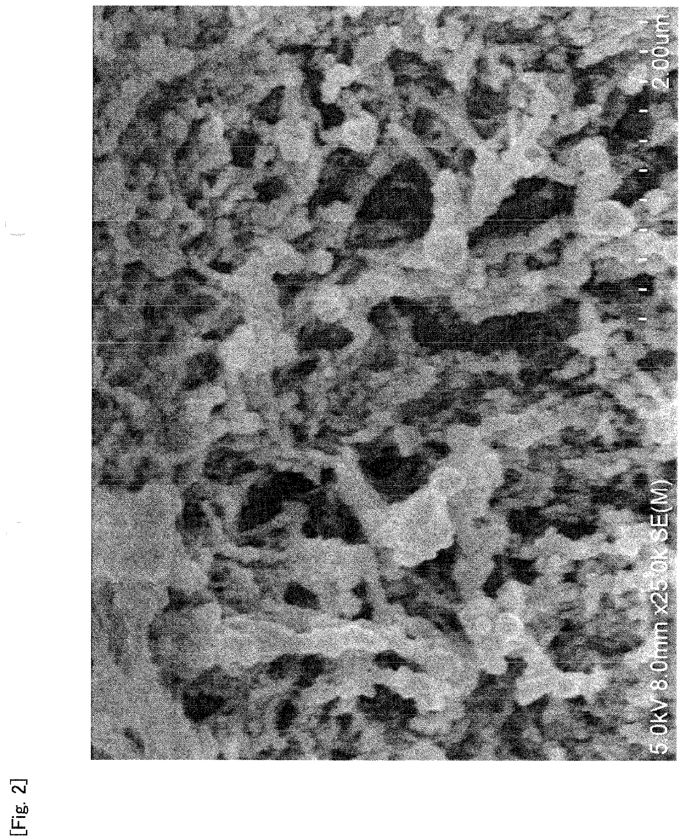

An adsorbent was prepared similarly to Example 1 except that a glycine aqueous solution as a water-soluble low molecular organic compound was not added during the preparation of a cellulose dispersion. The physical properties of the obtained adsorbent were evaluated. As a result, the amount of the immobilized Protein A was 35 g per 1 L of the adsorbent, and the 5% DBC of the adsorbent at RT of 3 min was 31 g per 1 L of the filling adsorbent. The SEM observation image of the beads surface is shown as Figure 2.

Example 2

(1) Epoxidation

The uncrosslinked cellulose beads prepared similarly to Example 1(3) were subjected to wet classification using sieves of 38 $\mu$m and 150 $\mu$m. To 1 time by volume of the classified beads, 1 time by volume of RO water was added, and 1.06 times by volume of 2N sodium hydroxide aqueous solution was further added. The mixture was heated at 45°C for 30 minutes. Then, 0.36 times by volume of epichlorohydrin was added thereto. The mixture was stirred at 45°C for 2 hours. The beads were filtered on a glass filter and washed with large amount of RO water to obtain epoxy group-containing porous cellulose beads. The amount of epoxy group to be contained was 34 pmol per 1 g of wet weight.

(2) Immobilization of dextran sulfate

To 0.7 times by volume of the epoxy group-containing porous cellulose beads, 26 wt/vol% aqueous solution of dextran sulfate (sulfur content amount: about 18%, molecular weight: about 4000) was added so that the liquid amount was adjusted to 1.0 time by volume. Then, 2N sodium hydroxide aqueous solution was added to adjust the pH value to 9.5. Next, the mixture was stirred at 40°C for 16 hours. The beads were filtered on a glass filter and washed with large amount of RO water to obtain the beads on which dextran sulfate was immobilized.

(3) Sealing of remaining epoxy group

To 1 time by volume of the dextran sulfate-immobilized beads, 1 time by volume of RO water and 0.25 times by volume of monoethanolamine were added. The mixture was stirred at 45°C for 2 hours to seal the remaining epoxy group. The beads were filtered on a glass filter and washed with large amount of RO water to obtain the target adsorbent.

The LDL cholesterol adsorption rate of the obtained adsorbent was measured by the method of Test example 4; as a result, the adsorption rate was 98.4% and the adsorption amount was 3.7 g/L per adsorbent volume.

Comparative example 2

An adsorbent was prepared similarly to Example 2 except that a glycine aqueous solution as a water-soluble low molecular organic compound was not added during the preparation of a cellulose dispersion. The LDL cholesterol adsorption rate of the obtained adsorbent was 91.9%, and the adsorption amount was 3.4 g/L per adsorbent volume.

Reference example 1

LDL cholesterol adsorption test was carried out similarly to Example 3 except that the adsorbent which filled an adsorption type plasma purification apparatus ("LIPOSORBER LA-15" manufactured by KANEKA CORPORATION) was used. As a result, the LDL cholesterol adsorption rate was 93.5%, and the adsorption amount was 3.5 g/L per adsorbent volume.

Example 3

(1) Preparation of porous cellulose beads

Porous cellulose beads were prepared similarly to the above-described Example 1(1) to (3). The obtained porous cellulose beads were classified to obtain porous cellulose beads having a particle diameter from 38 $\mu$m to 150 $\mu$m. After the liquid part of 100 mL of the obtained porous cellulose beads was replaced by ethanol, the beads were added into a reaction vessel and the total amount of the cellulose beads and ethanol was adjusted to 97 g. To the mixture, 28 g of distilled water and 80 mL of epichlorohydrin were added. The temperature of the solution was adjusted to 40°C and 96 mL of 1.8 N NaOH aqueous solution prepared from sodium hydroxide (manufactured by NACALAI TESQUE, INC.) and distilled water was added thereto to start a crosslinking reaction. After 1.5 hours from the start of the reaction, 9.6 mL of 17.0 N NaOH aqueous solution was added. In addition, after 3 hours and 4.5 hours from the start of the reaction, 9.6 mL of 17.0 N NaOH aqueous solutions were added. After 6 hours from the start of the reaction, the gel was separated and washed with distilled water of which volume was 20-fold of the volume of the beads.

The crosslinked cellulose beads obtained by the above-described crosslinking reaction were added into a reaction vessel, and the total amount of the cellulose beads and distilled water was adjusted to 116.7 g. After 37.8 g of sodium sulfate was added thereto and dissolved, 33 mL of epichlorohydrin was added and the mixture was maintained at 40°C. To the mixture, 21 mL of 17.0 N NaOH aqueous solution was added to start a crosslinking reaction. After 2.5 hours from the start of the reaction, 5 mL of 17.0 N NaOH aqueous solution was added. After 5 hours from the start of the reaction, the gel was separated and washed with distilled water of which volume was 20-fold of the volume of the beads.

(2) Measurement of $K_{av}$: gel distribution coefficient

In distilled water, 22.8 mL of the above-described porous cellulose beads were dispersed. The mixture was deaerated for 30 minutes. A column ("Tricorn 10/300" manufactured by GE healthcare Japan) was filled with the deaerated porous cellulose beads. The measurement was carried out using a size exclusion chromatography system (manufactured by SHIMADZU CORPORATION). The system contained DGU-20A3, RID-10A, LC-20AD, SIL-20AC and CTO-20AC, and LCSolution was used as a software.

The following dextran or glucose to be used as a marker was dissolved in 50 mM phosphate buffer (pH 7.5) containing 1M NaCl.

| Table 1 | | | |
|---|---|---|---|
| Molecular weight | Viscosity radius [nm] | Concentration [mg/mL] | Amount of injected marker [$\mu$L] |
| 1185000 | 27.0 | 3 | 40 |
| 667800 | 16.7 | 3 | 40 |
| 80900 | 6.8 | 1 | 80 |
| 48600 | 5.5 | 1 | 80 |
| 23800 | 3.9 | 1 | 80 |
| 11600 | 2.6 | 1 | 80 |

(continued)

| Table 1 | | | |
|---|---|---|---|
| Molecular weight | Viscosity radius [nm] | Concentration [mg/mL] | Amount of injected marker [μL] |
| 5220 | 1.8 | 1 | 80 |
| 180 | 0.4 | 10 | 40 |

While 50 mM phosphate buffer (pH 7.5) containing 1M NaCl was flowed through the column at a flow rate of 0.6 mL/min, a solution of dextran having a molecular weight of $4 \times 10^7$ was injected and the amount of liquid to be flowed through the column from the injection to the observation of the peak was measured by RI monitor in order to determine the volume except for the beads part in the column. The concentration of the solution of dextran having a molecular weight of $4 \times 10^7$ was adjusted to 10 mg/mL, and the injection amount was set to 40 μL. Then, the amount of each marker solution to be flowed through the column was similarly measured. The measurement values were plugged in the following formula to calculate the value of $K_{av}$.

$$K_{av} = (V_R - V_0) / (V_t - V_0)$$

wherein $V_R$ is the amount (mL) of a liquid to be flowed through the column from the injection of each marker solution to the observation of the peak, $V_0$ is the amount (mL) of a liquid to be flowed through the column from the injection of the solution of dextran having a molecular weight of $4 \times 10^7$ to the observation of the peak, $V_t$ is the volume (mL) of the beads in the column.

The result is shown in Table 2.

| Table 2 | | |
|---|---|---|
| Molecular weight | Viscosity radius [nm] | $K_{av}$ |
| 1185000 | 27.0 | 0.15 |
| 667800 | 16.7 | 0.22 |
| 80900 | 6.8 | 0.51 |
| 48600 | 5.5 | 0.56 |
| 23800 | 3.9 | 0.63 |
| 11600 | 2.6 | 0.69 |
| 5220 | 1.8 | 0.78 |
| 180 | 0.4 | 0.83 |

(3) Calculation of pore size distribution

The viscosity radius of each marker and the value of $K_{av}$ obtained as the above were plugged in the following formula in order to calculate the radius of the porous cellulose beads pore into which each marker was incorporated.

$$K_{av} = (1 - r_m / r_p)^2$$

wherein $r_m$ is the viscosity radius (nm) of each marker, $r_p$ is the radius (nm) of the pore into which each marker was incorporated.

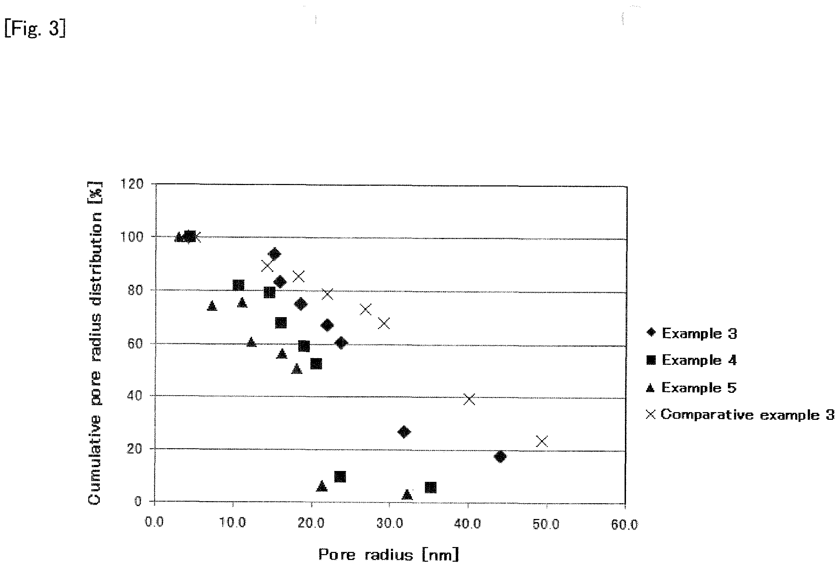

The graph of which abscissa is calculated pore radius of porous cellulose beads and of which ordinate is pore size distribution when the volume $(V_R - V_0)$ of the pore into which the marker having a molecular weight of 180 is incorporated is assumed to be 100% is shown as Figure 3.

Comparative example 3: Preparation of crosslinked porous cellulose beads without using water-soluble low molecular organic compound

(1) Preparation of cellulose dispersion

Into a separable flask, 103 g of distilled water and 9.3 g of cellulose were added. The mixture was stirred using two-stage rushton turbine blades at 150 to 200 rpm for 30 minutes till the temperature of the slurry became 4°C. Then, 41 g of 33 wt% sodium hydroxide aqueous solution which was cooled to 4°C was added thereto. The mixture was stirred at a rate of 500 rpm for 30 minutes.

(2) Preparation of porous cellulose beads by liquid-liquid dispersion

To the above-described cellulose dispersion, 788 g of 1 wt% sorbitan monooleate solution in o-dichlorobenzene was added. The mixture was stirred at 4°C and 600 rpm for 15 minutes to disperse cellulose droplets, and 74 mL of methanol as a coagulating solvent was added thereto. The mixture was stirred at 4°C and 600 rpm for 30 minutes. Then, the solution was removed by filtration using a glass filter ("26G-3" manufactured by TOP), and the cellulose beads were washed to be obtained using 5 times volume of methanol and 5 times volume of distilled water in turns.

(3) Measurement of gel distribution coefficient and calculation of pore size distribution

After the obtained porous cellulose beads were crosslinked in the condition similar to the above-described Example 3(1), the gel distribution coefficient was measured similarly to the above described Example 3(2) and the pore size distribution was calculated similarly to the above-described Example 3(3). The measurement result of $K_{av}$: gel distribution coefficient is shown in Table 3.

| Table 3 | | |
|---|---|---|
| Molecular weight | Viscosity radius [nm] | $K_{av}$ |
| 1185000 | 27.0 | 0.20 |
| 667800 | 16.7 | 0.34 |
| 80900 | 6.8 | 0.58 |
| 48600 | 5.5 | 0.63 |
| 23800 | 3.9 | 0.68 |
| 11600 | 2.6 | 0.73 |
| 5220 | 1.8 | 0.77 |
| 180 | 0.4 | 0.86 |

In addition, the graph of the pore size distribution with the result of the above-described Example 3 is shown as Figure 3. As Figure 3, it was confirmed the crosslinked porous cellulose beads of Comparative example 3 prepared without using a water-soluble low molecular organic compound showed a broad pore size distribution; on the one hand, the crosslinked porous cellulose beads of Example 3 prepared using a water-soluble low molecular organic compound showed a narrow pore size distribution.

Example 4

Porous cellulose beads were prepared similarly to the above-described Example 3 except that 6 wt% alanine aqueous solution was used in place of 6 wt% glycine aqueous solution.
With respect to the obtained porous cellulose beads, the gel distribution coefficient was measured similarly to the above described Example 3(2) and the pore size distribution was calculated similarly to the above-described Example 3(3). The measurement result of $K_{av}$: gel distribution coefficient is shown in Table 4.

| Table 4 | | |
|---|---|---|
| Molecular weight | Viscosity radius [nm] | $K_{av}$ |
| 1185000 | 27.0 | 0.05 |
| 667800 | 16.7 | 0.09 |
| 80900 | 6.8 | 0.44 |
| 48600 | 5.5 | 0.50 |
| 23800 | 3.9 | 0.57 |
| 11600 | 2.6 | 0.67 |
| 5220 | 1.8 | 0.69 |
| 180 | 0.4 | 0.85 |

In addition, the graph of the pore size distribution with the results of the above-described Example 3 and Comparative example 3 is shown as Figure 3. As Figure 3, the crosslinked porous cellulose beads of Example 4 prepared using alanine as a water-soluble low molecular organic compound showed a further narrow pore size distribution in comparison with the pore size distribution of Example 3.

Example 5

Porous cellulose beads were prepared similarly to the above-described Example 3 except that 9 wt% glycine aqueous solution was used in place of 6 wt% glycine aqueous solution.
With respect to the obtained porous cellulose beads, the gel distribution coefficient was measured similarly to the above described Example 3(2) and the pore size distribution was calculated similarly to the above-described Example 3(3). The measurement result of $K_{av}$: gel distribution coefficient is shown in Table 5.

| Table 5 | | |
|---|---|---|
| Molecular weight | Viscosity radius [nm] | $K_{av}$ |
| 1185000 | 27.0 | 0.03 |
| 667800 | 16.7 | 0.05 |
| 80900 | 6.8 | 0.39 |
| 48600 | 5.5 | 0.43 |
| 23800 | 3.9 | 0.46 |
| 11600 | 2.6 | 0.58 |
| 5220 | 1.8 | 0.57 |
| 180 | 0.4 | 0.76 |

In addition, the graph of the pore size distribution with the results of the above-described Example 3 and others is shown as Figure 3. As Figure 3, the crosslinked porous cellulose beads of Example 5 prepared using a water-soluble low molecular organic compound in higher concentration showed a much narrow pore size distribution.

Example 6

Porous cellulose beads were prepared similarly to the above-described Example 3 except that an amount of cellulose to be used was changed from 9.4 g to 7.7 g.
With respect to the obtained porous cellulose beads, the gel distribution coefficient was measured similarly to the above described Example 3(2) and the pore size distribution was calculated similarly to the above-described Example 3(3). The measurement result of $K_{av}$: gel distribution coefficient is shown in Table 6.

| Table 6 | | |
|---|---|---|
| Molecular weight | Viscosity radius [nm] | $K_{av}$ |
| 1185000 | 27.0 | 0.08 |
| 667800 | 16.7 | 0.09 |
| 80900 | 6.8 | 0.47 |
| 48600 | 5.5 | 0.51 |
| 23800 | 3.9 | 0.55 |
| 11600 | 2.6 | 0.70 |
| 5220 | 1.8 | 0.80 |
| 180 | 0.4 | 0.82 |

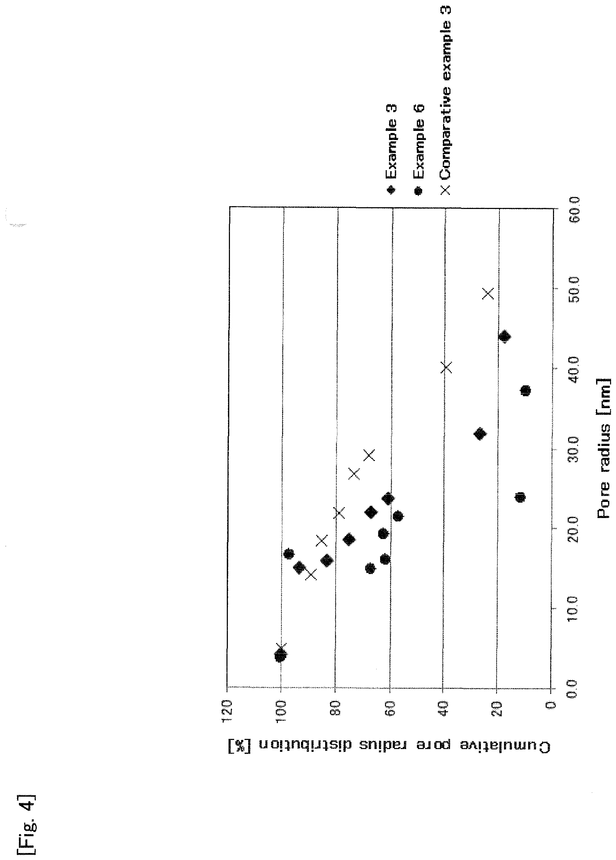

In addition, the graph of the pore size distribution with the results of the above-described Example 3 and Comparative example 3 is shown as Figure 4. As Figure 4, the crosslinked porous cellulose beads prepared by increasing the use ratio of a water-soluble low molecular organic compound to cellulose showed a much narrow pore size distribution.

**Claims**

1.  A method for producing porous cellulose beads, comprising

(a) the step of preparing a fine cellulose dispersion by mixing a low temperature alkaline aqueous solution and cellulose,

(b) the step of preparing a mixed liquid by adding a water-soluble low molecular organic compound to the fine cellulose dispersion,

(c) the step of preparing an emulsion by dispersing the mixed liquid in a dispersion medium,

(d) the step of contacting the emulsion with a coagulating solvent,

wherein the temperature of the alkaline aqueous solution in the step (a) is not less than -20°C and not more than 10°C; and

wherein the water-soluble low molecular organic compound is an amino acid.

2. The method for producing porous cellulose beads according to claim 1, wherein the water-soluble low molecular organic compound is one or more amino acids selected from the group consisting of glycine, alanine, serine, threonine, asparagine, glutamine, aspartic acid, glutamic acid, lysine, arginine and histidine.

3. The method for producing porous cellulose beads according to any one of claim 1 or 2, wherein the dispersion medium is one or more oil-soluble solvents selected from the group consisting of an animal and plant fat and oil, a hydrogenated animal and plant fat and oil, a fatty acid triglyceride, an aliphatic hydrocarbon solvent and an aromatic hydrocarbon.

4. The method for producing porous cellulose beads according to any one of claims 1 to 3, wherein the coagulating solvent is an alcohol solvent or a mixed solvent of water and an alcohol solvent.

5. A method for producing an adsorbent, comprising the step of producing porous cellulose beads by the method according to any one of claims 1 to 4, and

immobilizing a ligand capable of interacting with a target substance on the porous cellulose beads,

wherein the target substance is an antibody and the ligand is selected from an antigen, a protein and a peptide fragment which specifically interact with the antibody.

6. A method for purifying a target substance, comprising the steps of

producing an adsorbent by the method according to claim 5,

contacting a solution containing the target substance with the adsorbent,

separating the adsorbent on which the target substance is selectively adsorbed from the solution by filtration or centrifugation, and

separating the target substance from the adsorbent by using an eluate, or

comprising the steps of

producing an adsorbent by the method according to claim 5,

filling a column with the adsorbent,

flowing a solution containing the target substance through the column,

separating the target substance from the adsorbent by using an eluate.

**Patentansprüche**

1. Ein Verfahren zur Herstellung von porösen Celluloseperlen, umfassend

(a) den Schritt des Herstellens einer feinen Cellulosedispersion durch Mischen einer wässrigen Niedertemperatur-Alkalilösung und Cellulose,

(b) den Schritt des Herstellens einer gemischten Flüssigkeit durch Hinzugeben einer wasserlöslichen niedermolekularen organischen Verbindung zu der feinen Cellulosedispersion,

(c) den Schritt des Herstellens einer Emulsion durch Dispergieren der gemischten Flüssigkeit in einem Dispersionsmittel,

(d) den Schritt des Inkontaktbringens der Emulsion mit einem koagulierenden Lösungsmittel,

wobei die Temperatur der wässrigen Alkalilösung in Schritt (a) nicht weniger als -20°C und nicht mehr als 10°C beträgt; und

wobei die wasserlösliche niedermolekulare organische Verbindung eine Aminosäure ist.

2. Das Verfahren zur Herstellung von porösen Celluloseperlen gemäß Anspruch 1, wobei die wasserlösliche niedermolekulare organische Verbindung eine oder mehrere Aminosäuren ist, die aus der Gruppe bestehend aus Glycin, Alanin, Serin, Threonin, Asparagin, Glutamin, Asparaginsäure, Glutaminsäure, Lysin, Arginin und Histidin ausgewählt ist.

3. Das Verfahren zur Herstellung von porösen Cellulose-Perlen gemäß einem der Ansprüche 1 oder 2, wobei das Dispersionsmittel ein oder mehrere öllösliche Lösungsmittel ist, das aus der Gruppe bestehend aus einem tierischen und pflanzlichen Fett und Öl, einem hydrierten tierischen und pflanzlichen Fett und Öl, einem Fettsäuretriglycerid, einem aliphatischen Kohlenwasserstofflösungsmittel und einem aromatischen Kohlenwasserstoff ausgewählt ist.

4. Das Verfahren zur Herstellung von porösen Celluloseperlen gemäß einem der Ansprüche 1 bis 3, wobei das koagulierende Lösungsmittel ein Alkohollösungsmittel oder ein gemischtes Lösungsmittel aus Wasser und einem Alkohollösungsmittel ist.

5. Ein Verfahren zur Herstellung eines Adsorptionsmittels, umfassend die Schritte des Herstellens poröser Celluloseperlen mit dem Verfahren gemäß einem der Ansprüche 1 bis 4 und
Immobilisierens eines Liganden, der mit einer Zielsubstanz auf den porösen Celluloseperlen interagieren kann, wobei die Zielsubstanz ein Antikörper ist und der Ligand aus einem Antigen, einem Protein und einem Peptidfragment ausgewählt ist, die spezifisch mit dem Antikörper interagieren.

6. Ein Verfahren zur Reinigung einer Zielstubstanz, umfassend die Schritte des Herstellens eines Adsorptionsmittels mit einem Verfahren gemäß Anspruch 5,
Inkontaktbringens einer die Zielsubstanz enthaltenden Lösung mit dem Adsorptionsmittel,
Abtrennens des Adsorptionsmittels, an dem die Zielsubstanz selektiv aus der Lösung durch Filtration oder Zentrifugation adsorbiert wird, und
Abtrennens der Zielsubstanz von dem Adsorptionsmittel unter Verwendung eines Eluats, oder
umfassend die Schritte des
Herstellens eines Adsorptionsmittels mit einem Verfahren gemäß Anspruch 5, Befüllens einer Säule mit dem Adsorptionsmittel,
Fließens einer die Zielsubstanz enthaltenden Lösung durch die Säule,
Abtrennens der Zielsubstanz von dem Adsorptionsmittel unter Verwendung eines Eluats.

## Revendications

1. Méthode de production de billes de cellulose poreuses, comprenant

(a) une étape de préparation d'une dispersion fine de cellulose par mélange d'une solution aqueuse alcaline à basse température et de cellulose,
(b) une étape de préparation d'un liquide mélangé par addition d'un composé organique de faible masse moléculaire soluble dans l'eau à la dispersion fine de cellulose,
(c) une étape de préparation d'une émulsion par dispersion du liquide mélangé dans un milieu de dispersion,
(d) une étape de mise en contact de l'émulsion avec un solvant de coagulation,

dans laquelle la température de la solution aqueuse alcaline dans l'étape (a) n'est pas inférieure à -20°C et pas supérieure à 10°C ; et
dans laquelle le composé organique de faible masse moléculaire soluble dans l'eau est un acide aminé.

2. Méthode de production de billes de cellulose poreuses selon la revendication 1, dans laquelle le composé organique de faible masse moléculaire soluble dans l'eau est un ou plusieurs acides aminés choisis dans le groupe constitué par la glycine, l'alanine, la sérine, la thréonine, l'asparagine, la glutamine, l'acide aspartique, l'acide glutamique, la lysine, l'arginine et l'histidine.

3. Méthode de production de billes de cellulose poreuses selon l'une quelconque des revendications 1 ou 2, dans laquelle le milieu de dispersion est un ou plusieurs solvants solubles dans l'huile choisis dans le groupe constitué par les huiles et graisses animales et végétales, les huiles et graisses animales et végétales hydrogénées, un triglycéride d'acides gras, un solvant hydrocarboné aliphatique et un hydrocarbure aromatique.

4. Méthode de production de billes de cellulose poreuses selon l'une quelconque des revendications 1 à 3, dans laquelle le solvant de coagulation est un solvant alcoolique ou un mélange de solvants eau et solvant alcoolique.

5. Méthode de production d'un adsorbant, comprenant les étapes suivantes :

production de billes de cellulose poreuses par la méthode selon l'une quelconque des revendications 1 à 4, et immobilisation d'un ligand capable d'interagir avec une substance cible sur les billes de cellulose poreuses, dans laquelle la substance cible est un anticorps et le ligand est choisi parmi un antigène, une protéine et un fragment peptidique interagissant spécifiquement avec l'anticorps.

6. Méthode de purification d'une substance cible, comprenant les étapes suivantes :

production d'un adsorbant par la méthode selon la revendication 5,
mise en contact d'une solution contenant la substance cible avec l'adsorbant,
séparation de l'adsorbant sur lequel la substance cible est sélectivement adsorbée de la solution par filtration ou centrifugation, et
séparation de la substance cible de l'adsorbant par utilisation d'un éluat, ou
comprenant les étapes suivantes :

production d'un adsorbant par la méthode selon la revendication 5,
remplissage d'une colonne avec l'adsorbant,
circulation d'une solution contenant la substance cible à travers la colonne,
séparation de la substance cible d'avec l'adsorbant par utilisation d'un éluat.

[Fig. 1]

[Fig. 2]

[Fig. 3]

[Fig. 4]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2009242770 A
- WO 2006025371 A
- US 4634470 B
- US 5410034 B
- JP H9124702 A
- JP 2010236975 A
- CA 2828369 A1
- CN 101274985 A
- WO 2012121258 A

- WO 2008146906 A
- WO 2006004067 A
- US 5151350 A
- WO 2003080655 A
- JP 2006304633 A
- WO 2010110288 A
- JP H1278534 A
- EP 14854425 A
- JP 2013215120 A

### Non-patent literature cited in the description

- *Annals of the New York Academy of Sciences,* 2005, vol. 1051, 635-646
- *American Heart Journal,* 2006, vol. 152 (4), 712el-712e6

- *Journal of Chromatography,* 1980, vol. 195, 221-230
- **KENICHI KASAI et al.** Affinity chromatography. Tokyo Kagakudoujin, 1991